(19) Europäisches Patentamt
European Patent Office
Office européen des brevets

(11) Publication number: **0 117 651 A2**

# (12) EUROPEAN PATENT APPLICATION

(21) Application number: **84300583.6**

(51) Int. Cl.³: **C 07 D 501/18**

(22) Date of filing: **30.01.84**

(30) Priority: **31.01.83 EP 83400208**

(43) Date of publication of application: **05.09.84 Bulletin 84/36**

(84) Designated Contracting States: **AT BE CH DE FR GB IT LI LU NL SE**

(71) Applicant: **ICI PHARMA, 6 rue Blanche, F-95880 Enghien-les-Bains (FR)**

(72) Inventor: **Bruneau, Pierre, 9 rue des Vignes, F-51500 Ludes (FR)**

(74) Representative: **Brown, Ivor James Stewart et al, Imperial Chemical Industries PLC Legal Department: Patents PO Box 6, Welwyn Garden City Herts, AL7 1HD (GB)**

## (54) Process for the preparation of cephalosporins.

(57) A process for the manufacture of 7-(imidazol-2-yl) amino-3-(1H-1,2,3-triazol-4-yl)thiomethylceph-3-em-4-carboxylic acid, and the acid- and base-addition salts thereof, characterised by reaction of a compound of the formula I:

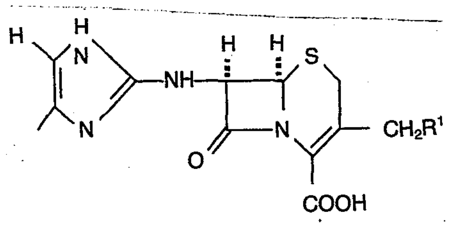

in which $R^1$ is a displaceable radical with 4-mercapto-1H-1,2,3-triazole or a base-additional salt thereof in a diluent or solvent consisting of trifluoroacetic acid, trichloroacetic acid, acetonitrile or sulpholane, or a mixture of any two or three of these, in the presence of boron trifluoride or a complex thereof;

whereafter when the product is obtained as the free base or the salt, and the salt or free base respectively is required, the interconversion between salt and free base is carried out by conventional means.

EP 0 117 651 A2

Z/PH. 32516/EP/F.

~~32/49~~



0117651

TITLE MODIFIED
see front page

PROCESS

This invention relates to a process for manufacturing a cephalosporin derivative.

European Patent Publications Nos. 31708 and 55562 describe 7-(imidazol-2-yl)aminocephalosporin derivatives carrying a variety of radicals at the 3-position. One of the methods disclosed therein for the manufacture of those compounds which carry a heterocyclylthiomethyl radical at the 3-position is the reaction of the corresponding 3-acetoxymethyl derivative with the appropriate heterocyclic thiol in a solvent in the presence of boron trifluoride etherate, following the general method described in UK Patent 1,565,941. It has been found, however, that this process gives poor yields of product when the required 3-substituent is (1H-1,2,3-triazol-4-yl)thiomethyl, and in particular when the object of the process is the compound 7-(imidazol-2-yl)amino-3-(1H-1,2,3-triazol-4-yl)thiomethylceph-3-em-4-carboxylic acid (European Patent Publication No. 31708, Example 19). It has now been found, however, that the above compound may be produced in high yield by operating the general process within controlled conditions.

According to the invention there is provided a process for the manufacture of 7-(imidazol-2-yl)amino-3-(1H-1,2,3-triazol-4-yl)thiomethylceph-3-em-4-carboxylic acid, and the acid- and base-addition salts thereof, characterised by reaction of a compound of the formula I:-

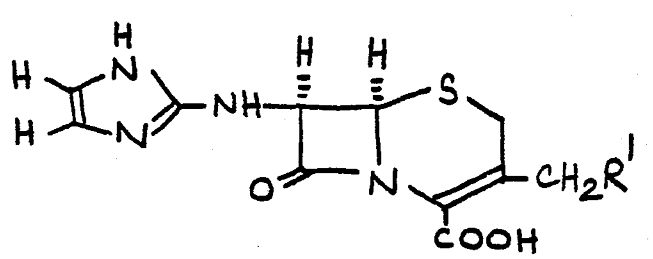

I

in which $R^1$ is a displaceable radical with 4-mercapto-1H-1,2,3-triazole or a base addition salt thereof in a

diluent or solvent consisting of trifluoroacetic acid, trichloroacetic acid, acetonitrile or sulpholane, or a mixture of any two or three of these, in the presence of boron trifluoride, or a complex thereof;

whereafter when the product is obtained as the free base or as a salt, and the salt or free base respectively is required, the interconversion between salt and free base is carried out by conventional means.

In the free base form the product of the process will generally exist in the form of the zwitterion. It will be noted that in the chemical name of the product manufactured by the process, and in formula I, the double bonds in the imidazole and triazole rings have been inserted in particular positions. It is to be understood, however, that other tautomeric forms of these heterocyclic residues are possible and these other forms are included within the scope of the process. In particular in the triazole ring the hydrogen atom may be attached to the 1-, 2- or 3-nitrogen atom.

A particular value for $R^1$ is 1-6C alkanoyloxy (e.g. acetoxy), chloro, bromo or iodo. The preferred value for $R^1$ is acetoxy.

A particular acid-addition salt of the cephalosporin derivative manufactured by the process of the invention is, for example, a salt formed with hydrochloric, hydrobromic, phosphoric, sulphuric, citric or maleic acid. A particular base-addition salt of the cephalosporin derivative manufactured by the process of the invention is, for example, an alkali metal salt (e.g. a sodium or potassium salt), an alkaline earth metal salt (e.g. a calcium or magnesium salt), or a salt with a primary, secondary or tertiary organic amine (e.g. triethylamine, procaine,

dibenzylamine and N,N[1]-dibenzylethylenediamine, and other amines which have been used to form salts with cephalosporins).

Though the 4-mercapto-1H-1,2,3-triazole may be used as such, in this form it has explosive properties, and it is preferably used in the form of a base-addition salt, e.g. the sodium salt. The free thiol may be liberated from this salt in situ. It is preferable to use equimolar amounts of the triazole and the compound of the formula I.

The boron trifluoride is preferably used in the form of a complex, e.g. a complex with a dialkyl ether such as diethyl ether, di-n-propyl ether or di-n-butyl ether, with an amine such as ethylamine, n-propylamine, isopropylamine, n-butylamine or triethanolamine, with an aliphatic acid such as acetic or propionic acid, with a nitrile such as acetonitrile or propionitrile, with a carboxylic ester such as methyl formate, ethyl formate or ethyl acetate, with a phenol such as phenol or with sulpholane. The preferred complex is that with sulpholane. It is preferable to use the boron trifluoride in excess, for example up to 20 fold excess. The preferred range is 5-12 fold excess.

The process may be carried out over the temperature range -10°C to 60°C, preferably in the range -5°C. to 40°C., and most preferably in the range -5°C. to 10°C.

Although the process of the invention will proceed under essentially anhydrous conditions, the rate of reaction is considerably accelerated by the presence of water. A suitable amount of water is in the range 0.3 to 5 molar equivalents of the compound of the formula I, and a preferred amount is in the range 1 to 2.5 molar equivalents.

When trichloroacetic acid or sulpholane is used as the sole solvent it is preferable, because of the melting points of these solvents (57-58°C. and 27.5°C. respectively), to use an additional diluent in order to achieve and maintain the fluidity and mobility of the reaction mixture at the operating temperature. A suitable diluent is methylene chloride, carbon tetrachloride, chloroform or diethyl ether. The preferred diluent for use with sulpholane is methylene chloride.

The reaction mixture may be worked up by conventional means, for example by drowning out the reaction mixture into a diluent in which the product is insoluble, followed by purification of the resulting precipitate by chromatography or by some other means. Alternatively the reaction mixture may be drowned out into water, the aqueous mixture optionally extracted with a water-immiscible organic solvent, and the product isolated from the aqueous solution by column chromatography.

The invention is illustrated, but not limited, by the following Examples. The yields quoted are to be regarded as illustrative rather than limiting. The temperatures are in degress Centigrade. The n.m.r. spectrum is quoted in delta relative to tetramethylsilane (delta = 0) as an internal standard (s = singlet, d = doublet, t = triplet, m = multiplet). The following contractions are used:-

| | | |
|---|---|---|
| TFA | - | trifluoroacetic acid |
| ether | - | diethyl ether |
| DMF | - | dimethylformamide |
| HOAc | - | acetic acid |
| DMSO | - | dimethylsulphoxide |

Example 1

To a solution of anhydrous 4-mercapto-1H-1,2,3-triazole monosodium salt (3 g.) in TFA (20 ml.) was added boron trifluoride diethyl etherate (8.0 ml.). The mixture was cooled to ambient temperature and a solution of 3-acetoxymethyl-7-(imidazol-2-yl)aminoceph-3-em-4-carboxylic acid (European Patent Publication 31708, Example 19; 2.0 g.) in TFA (12.0 ml.) was added in one portion. The mixture was stirred for two hours at ambient temperature and then added dropwise to ether (800 ml.) with stirring. The precipitate was collected by centrifugation and dried overnight in vacuo over KOH to give a dry powder (7.06 g.). A solution of this powder in a mixture of aqueous sodium acetate (0.1M; 90 ml.) and DMF (50 ml.) was purified by chromatography on AG1-X2 ion exchange resin (Biorad). The product was eluted with dilute aqueous HOAc (containing up to 2% v/v HOAc) to give, after evaporation and drying, 7-(imidazol-2-yl)amino-3-(1H-1,2,3-triazol-4-yl)thiomethylceph-3-em-4-carboxylic acid (1.53 g.) having the following n.m.r. in $d_6$DMSO:-3.5 (d, 1H); 3.8 (d, 1H); 4.0 (m, 2H); 5.1 (d, 1H); 5.5 (dd, 1H); 7.05 (s, 2H); 7.2 (m, 1H); 7.9 (s, 1H); 9.3 (d, 1H). The strength by HPLC was 90% giving an isolated yield of 61% (at 100% strength).

Example 2

3-Acetoxy-7-(imidazol-2-yl)aminoceph-3-em-4-carboxylic acid (0.96 g.) was added to molten sulpholane (16 ml.) at 29° and under an atmosphere of argon, followed by anhydrous 4-mercapto-1H-1,2,3-triazole monosodium salt (0.54 g.). TFA (1.0 ml.) was added to the resulting suspension, followed by boron trifluoride diethyl etherate (4 ml.). The resulting solution was stirred at ambient temperature for three hours and was then added to ether (400 ml.) with stirring. A gum was

formed, from which the ether was decanted. The gum was then dissolved in a mixture of aqueous sodium acetate (0.1 M; 66 ml.) and DMF (33 ml.), and was then purified by chromatography on AG1-X2 (acetate form) ion exchange resin (Biorad), eluting with dilute aqueous acetic acid (containing up to 0.5 % v/v HOAc) to give, after evaporation and freeze drying, 7-(imidazol-2-yl)amino-3-(1H-1,2,3-triazol-4-yl)thiomethylceph-3-em-4-carboxylic acid (0.85 g.) whose n.m.r. was identical to that given in Example 1. The strength by n.m.r. was 84.6% with 6.6% w/w water (Karl Fischer). This represents an isolated yield of 72% (at 100% strength).

Example 3

Anhydrous 4-mercapto-1H-1,2,3-triazole monosodium salt (0.54 g.) was added to acetonitrile under an atmosphere of argon. To the resulting suspension was added TFA (0.5 ml.) to form a clear solution. A suspension of 3-acetoxymethyl-7-(imidazol-2-yl)aminoceph-3-em-4-carboxylic acid (0.96 g.) in acetonitrile (10 ml.) was then added, followed by boron trifluoride diethyl etherate (4 ml.) to give a clear solution. This was stirred under argon for 3.5 hours at 23° and then the resulting mixture was poured into ether (400 ml.) with stirring. The solid which was formed was collected by filtration, washed with ether and dried *in vacuo* over KOH to give a solid (2.14 g.). This was dissolved and purified as described in the previous Example to give 7-(imidazol-2-yl)amino-3-(1H-1,2,3-triazol-4-yl)thiomethylceph-3-em-4-carboxylic acid (0.57 g.) whose n.m.r. was identical to that given in Example 1. The strength by HPLC was 83% with 7.2% w/w water (Karl Fischer). This represents an isolated yield of 47.8% (at 100% strength).

Example 4

Sulpholane/boron trifluoride complex (11.2 ml.; 57.0 m.mole) (25% w/w solution of boron trifluoride gas in sulpholane) was added dropwise over 2 minutes to anhydrous 4-mercapto-1H-1,2,3-triazole monosodium salt (687.5 mg.; 5.5 m.mole) under argon, maintaining the temperature of the mixture between 7° and 17° with ice cooling. The resulting suspension was stirred and cooled to 2° for 5 minutes, and then a suspension of 3-acetoxymethyl-7-(imidazol-2-yl)amino-ceph-3-em-4-carboxylic acid (1.845 g.; 5.0 m.mole) (91.9% strength by HPLC; 9.0% w/w water by Karl Fischer) in methylene chloride (9.8 ml.) was added in portions over 10 minutes, maintaining the temperature of the mixture below 12° throughout with ice cooling. The residual suspension was washed in with methylene chloride (2 x 2 ml.) and the resulting mixture was cooled to 2° and stirred for a further 130 minutes. After this time the reaction mixture was poured into ice/water (50 ml.). The residue from the flask was washed in with water (2 x 25 ml.) and the resulting solution was washed with methylene chloride (85 ml.). The yield of 7-(imidazol-2-yl)amino-3-(1H-1,2,3-triazol-4-yl)thiomethylceph-3-em-4-carboxylic acid, estimated by HPLC, was 1.722 g.(91%). The product was isolated by chromatography on Amberlite XAD-2 resin (Rohm and Haas), eluting with aqueous acetonitrile (containing 80% v/v acetonitrile, to give, after evaporation and freeze drying, 7-(imidazol-2-yl)-amino-3-(1H-1,2,3triazol-4-yl)thiomethylceph-3-em-4-carboxylic acid (1.714 g.) whose strength by HPLC was 84.1% with 4.9% w/w water (Karl Fischer). This represents an isolated yield of 76.1% (at 100% strength).

Claims

1. A process for the manufacture of 7-(imidazol-2-yl)amino-3-(1H-1,2,3-triazol-4-yl)thiomethylceph-3-em-4-carboxylic acid, and the acid- and base-addition salts thereof, characterised by reaction of a compound of the formula I:-

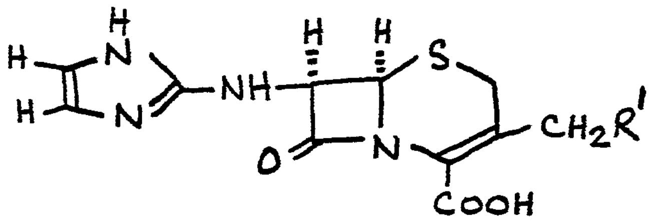

I

in which $R^1$ is a displaceable radical with 4-mercapto-1H-1,2,3-triazole or a base addition salt thereof in a diluent or solvent consisting of trifluoroacetic acid, trichloroacetic acid, acetonitrile or sulpholane, or a mixture of any two or three of these, in the presence of boron trifluoride, or a complex thereof;

whereafter when the product is obtained as the free base or as a salt, and the salt or free base respectively is required, the interconversion between salt and free base is carried out by conventional means.

2. A process as claimed in claim 1 in which $R^1$ is acetoxy.

3. A process as claimed in claim 1 or claim 2 which is carried out using the sodium salt of 4-mercapto-1H-1,2,3-triazole.

4. A process as claimed in claim 3 which is carried out in trifluoroacetic acid using boron trifluoride diethyl etherate at ambient temperature.

5. A process as claimed in claim 3 which is carried out in sulpholane/trifluoroacetic acid using boron trifluoride diethyl etherate at ambient temperature.

6. A process as claimed in claim 3 which is carried out in sulpholane/methylene chloride using boron trifluoride/sulpholane complex in the temperature range -5°C. to 10°C.

7. A process as claimed in claim 6 in which the boron trifluoride/sulpholane complex is present in 5 to 10 fold excess over the compound of the formula I.

8. A process as claimed in claim 7 in which the reaction mixture contains 1 to 2.5 molar equivalents of water.

IJSB/YB : 5 Dec.83
BS32575
SB25